# EUROPEAN PATENT APPLICATION

(11) **EP 4 257 067 A1**
(43) Date of publication of application: **11.10.2023**
(21) Application number: 22315079.8
(22) Date of filing: 07.04.2022
(51) Int. Cl.: A61B 18/14, A61B 18/00

(54) **APPARATUS AND METHOD FOR FORMING A RESTRICTION WITHIN AN ORGAN OF THE GASTROINTESTINAL TRACT**

(71) Applicant: BariaTek Medical, 75013 Paris (FR)
(72) Inventor: Biadillah, Joussef, 78600 Maisons-Lafitte (FR); Gray, Jonathan, 75011 Paris (FR); Naz, Christophe, 77300 Fontainebleau (FR); Pau, Antoine, 75017 Paris (FR)
(74) Representative: Hepp Wenger Ryffel AG

(57) **Abstract**

Apparatus for use in forming a restriction in an organ of the gastrointestinal tract, optionally, a stomach, the apparatus configured to be insertable into the interior of the organ, the apparatus comprising at least two, optionally at least three, optionally all of:
- an engagement device (20) for engaging and manipulating organ wall tissue, to displace the tissue, optionally by pulling and/or folding and/or rolling the tissue;
- a scarring device (30) for scarring tissue, optionally displaced tissue;
- a device for delivering a spacer (40) and/or separator to tissue of the organ wall, optionally to displaced and/or engaged tissue, and/or tissue within operative range of the scarring device;
- a fixing device for inserting one or more tissue fixings optionally at a scarred zone of tissue.

## Description

### Field of the Invention

In one non-limiting aspect, the present invention relates to the formation within an organ of the gastrointestinal tract, of a restriction. The organ may be the stomach.

### Background to the Invention

Bariatric diseases represent a major problem in present day society, with 13% of the worldwide population being obese and 39% overweight. Bariatric surgery is one of the most promising means of treating these problematic diseases when other courses of treatment fail.

Even though considerable progress in the field of bariatric surgery has been made, there still remain many complicated problems that need addressing to provide satisfactory and durable results for the patients.

For example, gastric sleeve surgery is well known for helping reduce the patient's weight, but said weight loss is in most cases only temporary due to the expandable nature of the stomach tissue. Therefore, this only allows for a temporary solution and may lead to patients regaining weight in the long-term. Furthermore, gastric sleeve surgery is an extremely invasive procedure wherein the majority of the stomach is surgically removed, leading to extensive recovery time. Less-invasive endoscopic procedures have been proposed, but these are generally less durable and less effective effect than surgical procedures.

US 2017 020 592 A1 proposes a surgical procedure to be performed posteriorly to an unsuccessful bariatric weight loss surgery and consisting in ablating tissue at the surgical site of a previous operation.

US 2017 367 723 A refers to a method of ablating ghrelin producing cells so as to reduce the production of the hunger hormone ghrelin. More specifically the method pertains to the cellular death to the A-X type cells in a patient's stomach.

### Summary of the invention

The present invention aims to improve patient outcome and versatility in treatments and devices available for sustainable weight loss in obese patients.

A first aspect of the invention provides an apparatus for use in forming a restriction in an organ of the gastrointestinal tract. The organ may optionally be a stomach. The apparatus may be insertable into the interior of the organ, optionally through a natural body opening.

The apparatus may comprise a device for displacing a region of tissue of the organ wall from within the organ to modify the shape of the organ wall.

The apparatus may further comprise a device for scarring tissue in a target zone at or adjacent to the displaced tissue region to alter tissue wall properties in the target zone, to effect and/or to support the restriction.

The term scarring is to be understood as the deliberate wounding of tissue so that there may be formation or appearance of a scar or of multiple scars on the tissue after the wound has healed.

The engaging device may be configured for insertion within a patient, optionally through a natural orifice of the patient. The engaging device may carry a scarring device for scarring tissue in a target zone.

Furthermore, the engaging device may comprise one or more engaging ports for allowing the operation of one or more engaging devices and/or scarring devices.

In some embodiments the engaging device may be configured to engage with a portion of an organ wall, from within the organ, for inwardly and/or externally drawing and/or restricting a portion of the organ wall. Optionally the engaging device may be configured to pull at least a portion of an organ wall (e.g. towards the engaging device) for changing the position of the portion of wall. e.g. inwardly and/or outwardly.

The engaging device may allow the distancing of the organ wall from surrounding tissue. It may further allow the manipulation of the organ into a desired configuration or shape. This advantage may further help avoid damage to non-targeted tissue during a procedure. In some embodiments, a spacer may optionally be provided to space and/or separate targeted tissue from non-targeted tissue, also to help avoid damage to non-targeted tissue. More details of such a spacer are described later below.

In some embodiments the engaging device could provide an engaging element, or engaging elements, for displacing the portion of organ wall. Optionally the engaging element(s) may operate through one or more engaging ports of the engaging device.

The engaging element may, for example, comprise a selection of one or more suction elements for drawing a portion of organ wall by negative pressure; one or more helical screws for screwing into the organ wall; clasps for clasping the organ wall.

In some embodiments the engaging device may be further configured to at least partly rotate for forming a restriction of the organ.

Optionally, rotation of the engagement device may induce a rotation of the engaged portion of organ wall. The rotation may perform an at least partly rolled, folding of the organ wall. Further optionally, the rotation may induce rolling of the organ wall around the engaging device. This may allow restriction of the size of the organ and/or distancing the organ, or a portion of the organ, from surrounding tissue.

The engaging device may, for example, rotate through at least 180°, optionally at least 270°, optionally at least 360°, optionally at least 720° to roll or coil the portion of grasped tissue. The engaging device may perform a, e.g., up to 720° or more rotation for reducing the size of the organ.

The device may be configured to displace at least a portion of organ wall according to, but not limited to, any combination of the above embodiments.

The invention may further provide a device for scarring tissue in a target zone to alter tissue wall properties in the target zone. The target zone may be at, or adjacent, to the displaced tissue region. This may help effect and/or to support the restriction of the organ.

The scarring device may be configured to damage the organ wall in such a manner that scar tissue formation may be promoted.

Scar tissue is generally defined as the tissue formed when a wound is repaired. Scar tissue is also defined as having different properties than normal tissue, for example, scar tissue is harder and stiffer than normal tissue, and scar tissue may also contract over time.

Additionally or alternatively, the scarring device may scar a plurality of portions of organ wall in close proximity of one another. This may induce the formation of one or more adhesions between the plurality of portions of organ wall.

Adhesions are understood to be bands of scar-like tissue that forms between two surfaces inside the body and cause them to stick together.

Promoting formation of scar tissue and/or adhesions of the organ wall may therefore help reduce the motility of the organ wall as well as possibly helping secure the organ in a restricted configuration.

In some embodiments the scarring device may be configured to thermally damage the organ wall. For example, the scarring device may use caustic and/or cautery agents.

Optionally the scarring device may induce thermal damage by means of laser and/or radiofrequency treatment to the organ wall.

In some embodiments the scarring device may be operated subsequently to and/or simultaneously with operation of the engaging device. Additionally or alternatively, the scarring device and the engaging device may be operated in an alternating manner.

In some embodiments it may be desirable to scar one or more zones of the portion of displaced tissue. Additionally or alternatively, it may be desirable to scar a different portion of organ wall to the portion that may have been displaced.

Scarring one or more zones of the organ wall may reduce the motility of the organ. Furthermore, it may also promote shrinkage of the organ or fusion of a plurality of portions of the organ wall. Damaging the organ wall once the engaging device has been operated may avoid collateral damage, for example, to surrounding non-targeted tissues.

In a second aspect the invention provides, optionally in combination with the first aspect, a fixation device for placing one or more tissue engaging and/or tissue penetrating fixations. Optionally the fixations are placed in a zone of tissue damaged by the or a tissue scarring device.

The fixations may be configured to secure the organ in a reduced size. Optionally the fixations may secure a rolled fold of the organ. Placing the fixations in a damaged zone of organ wall may avoid excessive tension on the fixations and therefore may help to avoid the dislodging or breakage of the fixations.

The fixations may be any suitable means for securing the organ in a reduced size. For example, the fixations may be sutures; pledgets; staples.

The reduced mobility of the scar tissue, optionally combined with the placing of fixations, may avoid the post-procedural expanding of the organ and therefore may allow for a more durable procedure for treating obesity.

In some embodiments and according to any of the aspects of the invention, the engaging device may be further configured to disengage the portion of engaged organ wall to facilitate the extraction of the apparatus from within the patient. For example, the engaging device may disengage the grasped portion of tissue by applying of positive pressure. Optionally, the use of positive pressure may distance the organ wall from the device and therefore facilitate the extraction. Also, for example, the engagement device may disengage the portion of organ wall by the unscrewing of helical screws.

Additionally or alternatively, the engaging device may be at least partly collapsible for disengaging with the organ wall. This may facilitate removal of the apparatus from within the patient.

The engaging device may have a first operational configuration for repositioning and/or damaging the organ wall and a second collapsed configuration for insertion and/or removal of the apparatus from within the patient.

The apparatus as provided by the invention may be configured to promote scarring of at least a zone of an organ wall for reducing the size and/or the motility of the organ.

In a third aspect the invention provides a method of forming a restriction in an organ of the gastrointestinal tract. The organ may optionally be a stomach. The method may optionally use a device as described above.

The method may comprise a step of inserting an engaging device within a patient, optionally through a natural body orifice.

The method may include a step consisting of actuating an engaging device to modify the shape of the organ, for example, by displacing organ wall tissue. In some embodiments actuating the engaging device may modify the shape of the organ by pulling and/or pushing a portion of the organ wall. Optionally, the engaging device may be actuated to inwardly draw a portion of the organ wall.

Additionally or alternatively, the method may provide a step of actuating the engaging device for manipulating the organ wall tissue into roll-like fold.

Additionally or alternatively, the method may provide a step of deploying a spacer in the region of the displaced organ wall tissue, to space and/or separate the displaced organ wall tissue from surrounding tissue.

Additionally or alternatively, the method may provide a step of operating a scarring device to alter at least a zone of organ wall tissue, optionally altering an adjacent target zone to.the portion of organ wall tissue engaged by the engaging device.

Optionally the steps for actuating the engaging device and for actuating the scarring device may be performed simultaneously with each other, or one subsequently after the other, or in an alternating sequence.

Further additionally or alternatively, the method may provide a step of actuating a fixation device for placing one or more fixation elements in a damaged target zone of organ wall tissue.

A fourth aspect of the invention, optionally in combination with any of the preceding aspects, provides at least two, optionally all three of:
- an engagement device for engaging and manipulating organ wall tissue, to displace the tissue (optionally by pulling and/or folding and/or rolling the tissue);
- a scarring device for scarring tissue (optionally displaced tissue);
- a fixing device for inserting one or more tissue fixings (optionally at a scarred zone of tissue).

The fourth aspect specifically envisages: in one form at least the engagement device and the scarring device; and in another form at least the scarring device and the fixing device.

A fifth aspect of the invention, optionally in combination with any of the preceding aspects, provides a method comprising at-least two, optionally all three of:
- an engagement step of engaging and manipulating organ wall tissue, to displace the tissue (optionally by pulling and/or folding and/or rolling the tissue);
- a scarring step of scarring tissue (optionally displaced tissue);
- a fixing step of inserting one or more tissue fixings (optionally at a scarred zone of tissue).

The fifth aspect specifically envisages: in one form at least the engagement step and the scarring step; and in another form at least the scarring step and the fixing step.

In a sixth aspect of the invention, optionally in combination with any of the preceding aspects, the apparatus may comprise or further comprise a device for delivering a spacer to an engaged and/or displaced portion of organ wall. Optionally, the device for delivering a spacer may be integrated within the engaging device.

The spacer may be delivered in such a manner that it at least partly circumscribes the engaged portion of organ wall. For example, the spacer may be configured to be placed, at least temporarily, around an engaged portion of organ wall in such a manner that two sides of the engaged portion of wall are brought and/or maintained at least partly closer together.

The spacer may be any suitable means for creating a separation between a target tissue and a non-target tissue in such a manner that the use of a scarring device on targeted tissue does not affect non-targeted tissue. For example, the spacer may be a thermal separator to obstruct transmission of thermal energy to non-targeted tissue.

In some embodiments, the thermal separator may be delivered to an engaged portion of an organ wall through a port of the engaging device. Alternatively, it may be delivered to an engaged portion of organ wall within the engaging device. For example, if the potion of organ wall is pulled within the engaging device by means of an engaging element.

For example, the thermal separator may be placed before the use of a scarring device, optionally a scarring device configured to thermally damage an organ wall.

The thermal separator may prevent a non-targeted tissue from damage due to the use of caustic and/or cautery agents.

Additionally or alternatively, the thermal separator may prevent transmission of laser and/or radiofrequency treatments to non-targeted tissue.

In some embodiments, the thermal separator may be a cooling balloon configured to avoid dissipation of thermal energy to non-targeted tissue. The cooling balloon may be at least partly toroidal for circumscribing the engaged and/or displaced portion of organ wall. The balloon may be configured to contain and/or circulate a fluid, preferably a cooling fluid for preventing the transmission of a thermal energy to non-targeted tissue.

Additionally or alternatively, the separator may be inflatable, to increase the physical separation distance when inflated. The inflation fluid may, for example, be a coolant fluid as described above.

The thermal separator provides the advantage of providing an insulating separation between a target tissue and non-targeted tissue, and thus may reduce the risk of unintentional damage to surrounding tissue.

Although certain features and aspects have been outlined above, this does not limit the scope of protection. Protection is claimed for any novel feature and/or idea disclosed herein and/or illustrated in the drawings whether or not emphasis has been placed thereon.

### Brief description of the drawings

Non-limiting embodiments are now described by way of example only, with reference to the accompanying drawings, in which:
Fig. 1 is a schematic perspective view of an embodiment of the apparatus for damaging the gastrointestinal tract;
Figs. 2a-d are schematic representations illustrating use of the first embodiment of apparatus for damaging the gastrointestinal tract;
Fig. 3 is a schematic flow diagram of a first general method of use;
Fig. 4 is a schematic flow diagram of a second general method of use;
Fig. 5 is a schematic flow diagram of a third general method of use.
Fig. 6a-h are schematic representations illustrating use of a second embodiment of the apparatus for damaging the gastrointestinal tract.
Fig. 7a-f are schematic representations illustrating use of a third embodiment of the apparatus for damaging the gastrointestinal tract.
Fig. 8 is a schematic flow diagram of a fourth general method of use.

### Detailed description of the drawings:

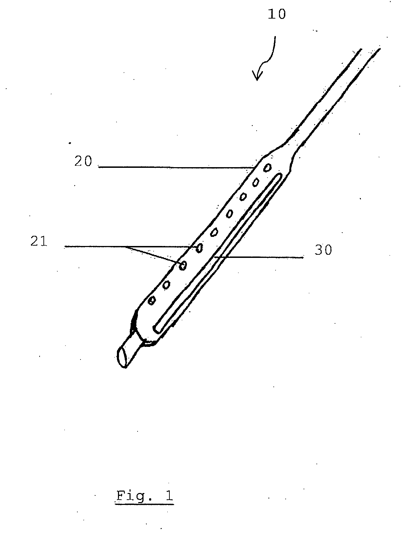

Referring to fig. 1, an apparatus 10 provides an instrument body 11 configured to be inserted within a patient and comprising a working region 20 (e.g. working end) provided with a plurality of engaging devices 21 and a scarring device 30. The engaging devices 21 are operable to cause engagement between the working end 20 and a portion of an organ wall. For example, the engaging devices 21 may be or comprise suction ports for application of negative pressure to suck tissue against the body 11, or other positive engagement,devices. The one or more engagement devices 21 may be provided entirely within the working end 20, or a portion of the one or more engagement devices 21 may be provided elsewhere. For example, a negative pressure generator may be provided or coupled to the apparatus outside the body.

The scarring device 30 is configured to damage and/or scar at least a zone of an organ wall. For example, the scarring device may comprise a device for applying to the tissue, or generating within the tissue, energy, for example, heat to thermally damage the tissue. The scarring device may comprise a laser and/or an RF electrode and/or argon plasma. The laser and/or RF energy and/or argon plasma may be generated within a portion of the instrument within the body, or supplied from outside the body to the instrument working end 20. Although the scarring device 30 is illustrated as being part of the same instrument as the engagement device(s) 21, in other embodiments, the scarring device 30 may be a separate or separately introducible instrument.

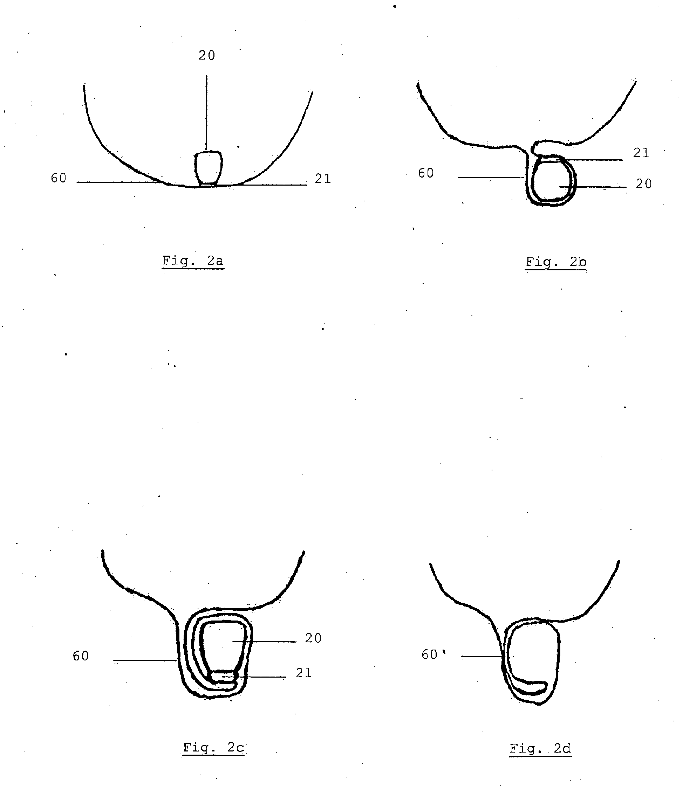

Referring to figs. 2a-d, in one example method of use, the working end 20 of the instrument 10 is inserted within an organ 60, for example, of the gastro-intestinal tract. The organ 60 may be stomach. The instrument 10 may be inserted through a natural body orifice, for example the mouth, to access the organ 60.

The instrument 10 is manipulated to bring the working end 20 into engagement with a portion of the organ wall tissue by means of the one or more engagement devices 21 (see fig. 2a). In a first rotation step, the working end 20 is rotated, for example, by 360° thereby rolling the tissue of the engaged organ wall (see fig. 2b). In a further step, the working end 20 is further rotated to 720° and produces a rolled-up folding of the organ wall (see fig. 2c). In the illustrated example, the tissue is rolled outwardly, to create a fold or bulge on the exterior of the organ, but in other embodiments, the tissue could be rolled inwardly to create a fold or bulge within the interior of the organ.

At any one or more stages during the rotation/rolling, the scarring device 30 can be operated to the generate scarring in the tissue wall. For example, the scarring device 30 may be operated after the first rolling step and/or after the second rolling step. Alternatively, each rolling step may be performed as a sequence of multiple sub-steps, and the scarring device 30 may be operated after each sub-step.

In fig. 2d the engagement device is disengaged and the working end 20 removed from rolled, folded tissue. In one form, positive pressure may be applied through the ports previous used as suction ports, in order to positively free the working end 20 from the tissue wrapped around the working end. In another form, the working end 20 may be at least partly radially collapsible to provide additional space for freeing the working end 20 from the tissue wrapped around the working end.

The above method provides numerous advantages for forming a size restriction in the organ 60. Scarring the tissue can shrink the tissue, and hence reduce organ volume. Scarring can also reduce motility of the tissue. In the case of a stomach organ 60, scarring can also reduce the creation of gastric juices by the damaged tissue, and hence contribute desirably to reduced stomach function.

The one or more engagement devices 21 enable the tissue to be rolled to reduce the organ volume in addition to, or as an alternative to, shrinkage by scarring. Additionally or alternatively, the one or more engagement devices enables the organ wall tissue to be pulled clear of other neighbouring body organs, thereby significantly reducing risk of accidental damage to other organs by operation of the scarring device 30.

Although not shown explicitly, the apparatus may further comprise a fixation device for applying one or more tissue-penetrating fixings to the rolled tissue. The fixation device may form part of the working end 20 of the instrument 11, or it may be a separate device introducible to the tissue after removal of the instrument working end 20. The tissue-penetration fixings may secure the scarred tissue in its rolled form. Example tissue-penetrating fixings include one or more sutures, or one or more pledgets, or one or more insertable anchors.

The scarred tissue, having reduced motility, and reduced regeneration capacity, can provide a stable environment for secure attachment of the fixings. Such tissue can have a significantly reduced tendency, compared to undamaged tissue, to release or expel fixings that are embedded within the tissue.

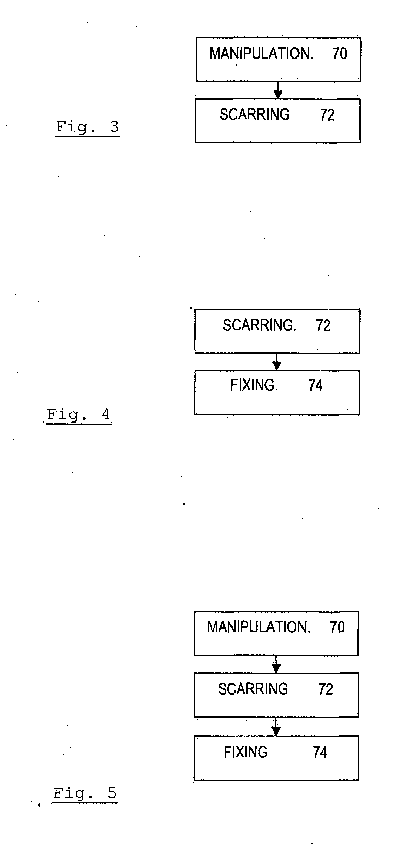

By way of example, Figs. 3 to 5 illustrate general methods described herein.

Fig. 3 illustrates a first example method including a manipulation step 70 of engaging and manipulating organ wall tissue, from within the organ, to displace the tissue, for example, by pulling and/or rolling and/or folding the tissue. A scarring step 72 comprises using a scarring device 30 to scar the tissue, for example, to scar a region of the tissue that has been displaced or an adjacent region. The method may be repeated several times to progressively displace and scar organ wall tissue.

Fig. 4 illustrates a second example method including a scarring step 72, to intentionally damage organ wall tissue. A fixing step 74 comprises applying one or more tissue penetrating fixings to the scarred tissue.

Fig. 5 illustrates a third example method including the manipulation step 70 as described above, the scarring step 72 described above, and the fixing step 74 described above, and combining all of the results and advantages of the first and second methods.

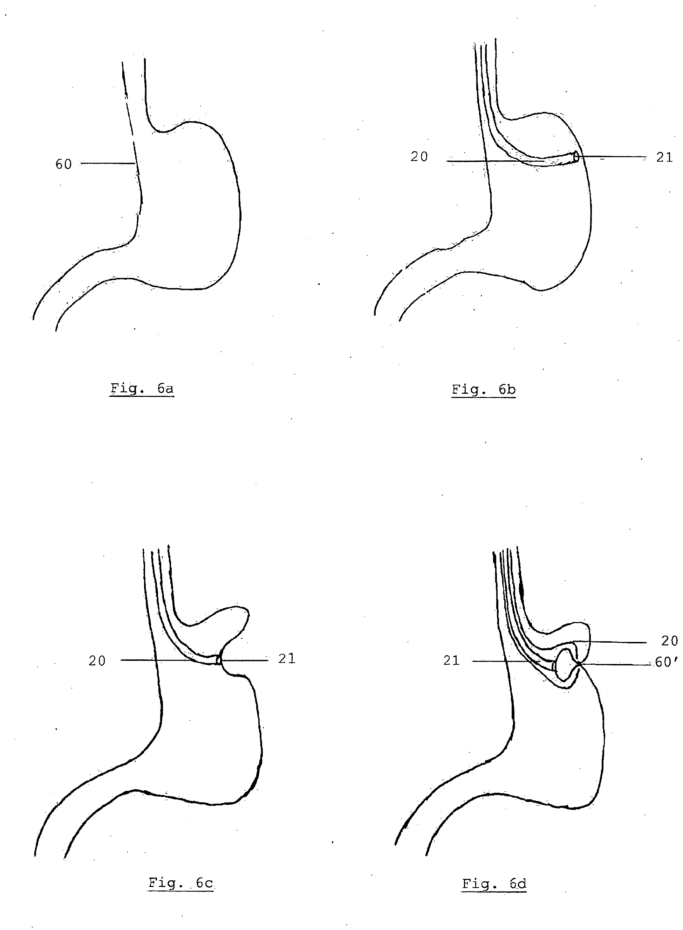

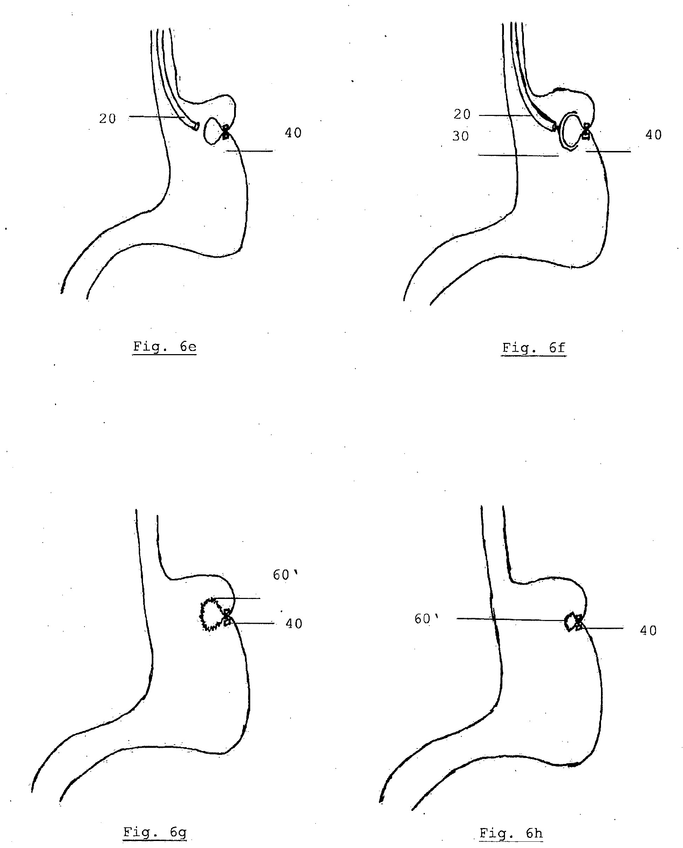

Referring to figs. 6a-h, in one example method of use, the working end 20 of the instrument 10 is inserted within an organ 60, for example, of the gastro-intestinal tract. Optionally, the instrument 10 is inserted through a natural body orifice, for example the mouth. The organ 60 may be stomach.

In figs. 6b-d, the instrument 10 is manipulated to bring the working end 20 into engagement with a portion of the organ wall tissue by means of the one or more engagement devices 21. The instrument 10 is manipulated to pull on the engaged tissue, to form a bulge into the interior of the organ. A delivery device is operated to deliver a spacer and/or separator 40 (referred to hereafter as spacer/separator) to the engaged portion of organ wall. The spacer/separator 40 is delivered so as to at least partly circumscribe the engaged portion of tissue and bringing, or maintain, two sides of an engaged tissue closer to one another.

The delivery device may form part of the working end 20 of the instrument 11, or it may be a separate device introducible to the tissue.

As seen in fig. 6e, the presence of the spacer/separator 40 maintains the engaged portion of tissue in an at least partly bulb-like configuration. In the illustrated embodiment, the spacer/separator can be a thermal separator, to provide a thermal barrier that protects tissue behind the thermal separator from damage during the scarring procedure. A thermal separator may, for example, be provided by heat insulating material, and/or a heatsinking material, and/or a cooled device (for example, cooled by circulation of a fluid coolant, also illustrated in the next embodiment).

In the illustrated embodiment, the spacer/separator 40 may remain in place on the tissue after the procedure, for example, to act as a tissue fixation device. Alternatively (and as illustrated in more detail in the next embodiment, the spacer/separator 40 may be removed after the procedure).

Referring to figs. 6f-h the scarring device 30 is operated to the generate scarring in the tissue wall. The scarring device 30 is configured to adapt to the configuration of the targeted tissue. For example, as seen in fig. 6f, the scarring device 30 adopts the generally bulb-like shape of the targeted tissue secured by the separator 40. Due to the separator 40 the damage from the scarring device 30 is limited to the target tissue.

Seen in fig. 6h once healed the tissue damaged by the scarring device has healed and contracted, thus leading to a restriction of the organ.

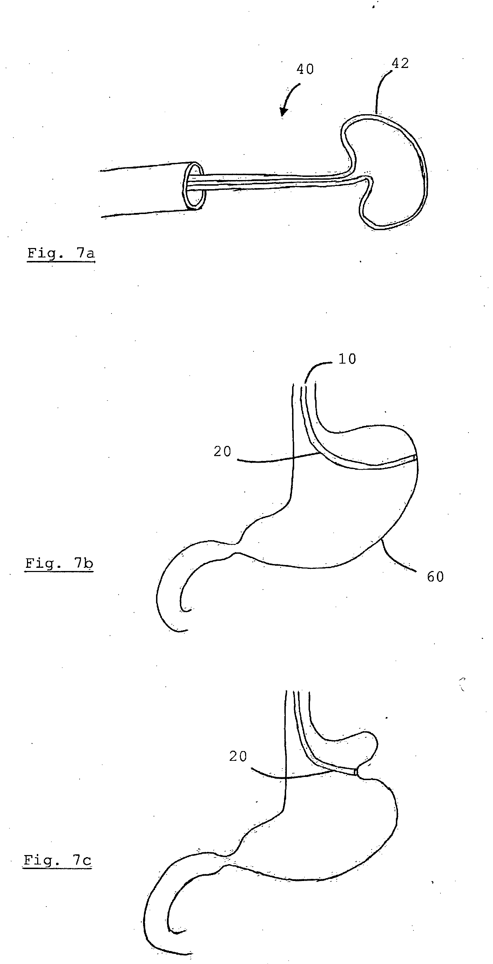

Figs. 7a-f illustrate a further example method of use similar to Figs. 6a-h, employing a spacer and/or separator (spacer/separator) 40. In this example, the spacer/separator 40 is placed temporarily, and is removed after the procedure. Referring to Fig. 7a, the spacer/separator 40 comprises a conduit 42 defining a loop. In one form, the conduit permits a coolant fluid to be circulated to actively cool adjacent tissue by absorbing and removing heat via the circulated coolant. In another form, the conduit 42 permits the spacer to be inflated and/or distended to enlarge the conduit into a doughnut shape. In another form, the conduit can be both actively cooled, and inflated, for example, by using a pressurized coolant fluid.

Referring to fig. 7b, and similarly to the preceding embodiment, the working end 20 of the instrument 10 is inserted within an organ 60, for example, of the gastro-intestinal tract. Optionally, the instrument 10 is inserted through a natural body orifice, for example the mouth. The organ 60 may be stomach. The instrument 10 is manipulated to bring the working end 20 into engagement with a portion of the organ wall tissue by means of the one or more engagement devices 21.

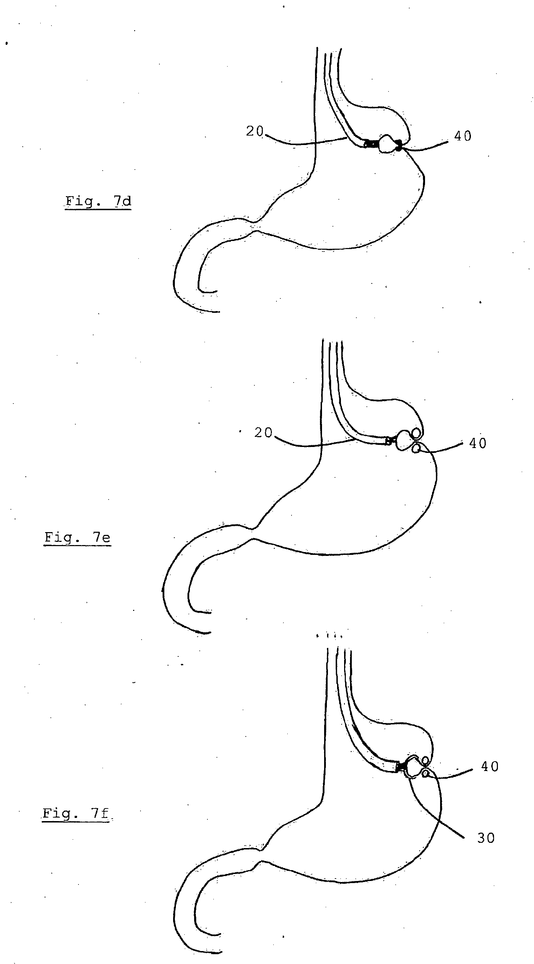

Referring to Fig. 7c, the instrument 10 is manipulated to pull on the engaged tissue, to form a bulge into the interior of the organ. Referring to Fig. 7d, a delivery device is operated to deliver the spacer/separator 40 to the engaged portion of organ wall. The spacer/separator 40 is delivered so as to at least partly circumscribe the engaged portion of tissue and bring, or maintain, two sides of an engaged tissue closer to one another. The delivery device may form part of the working end 20 of the instrument 11, or it may be a separate device introducible to the tissue. The spacer/separator 40 may be extendable/retractable with respect to the delivery device, to fit around the displaced tissue and to constrict the tissue to form the bulb shape.

As seen in fig. 7d, the presence of the spacer/separator 40 maintains the engaged portion of tissue in an at least partly bulb-like configuration. In the illustrated embodiment, the spacer/separator can be a thermal separator, to provide a thermal barrier that protects tissue behind the thermal separator from damage during the scarring procedure. As described above, the loop shape permits circulation of a coolant fluid and/or inflation into a doughnut form to increase the physical spacing of the bulb from the tissue behind the spacer/separator 40, as illustrated in Fig. 7e

Referring to fig. 7f, the scarring device 30 is operated to the generate scarring in the tissue wall. The scarring device 30 is configured to adapt to the configuration of the targeted tissue. For example, as seen in fig. 7f, the scarring device 30 adopts the generally bulb-like shape of the targeted tissue secured by the separator 40. Due to the separator 40 the damage from the scarring device 30 is limited to the target tissue. The spacer/separator 40 can be removed after the procedure, for example, by extending the loop with respect to the delivery device, which expands the size of the loop and allows unhindered removal from the tissue.

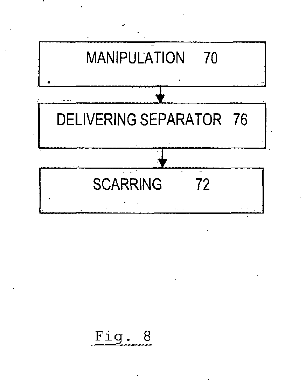

Fig. 8 illustrates a fourth example method comprising the manipulation step 70 as described above, a delivering step 76 for delivering a spacer/separator to an engaged and/or displaced tissue and a scarring step 72 as described above.

It will be appreciated that the foregoing description is merely illustrative of examples of the invention, and that many modifications and equivalents may be made within the scope and/or principles of the invention.

## Claims

1. Apparatus for use in forming a restriction in an organ of the gastrointestinal tract, optionally a stomach,, the apparatus configured to be insertable into the interior of the organ, the apparatus comprising:
a device for displacing a region of tissue of the organ wall from within the organ to modify the shape of the organ wall; and
a device for scarring tissue in a target zone at or adjacent to the displaced tissue region to alter tissue wall properties in the target zone.

2. Apparatus according to claim 1, wherein the device for scarring tissue is configured to scar tissue to effect and/or to support the restriction.

3. Apparatus according to claim 1 or 2, wherein the engaging device is configured to pull at least a portion of the organ wall.

4. Apparatus according to any of claims 1 to 3, further comprising a device for delivering a spacer and/or separator to an engaged and/or displaced portion of organ wall, the spacer configured to protect adjacent tissue from the scarring device.

5. Apparatus according to claim 4, wherein the spacer is a thermal spacer.

6. Apparatus optionally according to any of claims 1 to 5, the apparatus configured for use in forming a restriction in an organ of the gastrointestinal tract, optionally a stomach, the apparatus configured to be insertable into the interior of the organ, the apparatus comprising:
a fixation device for placing one or more tissue engaging and/or tissue penetrating fixations in a zone of scarred tissue.

7. Apparatus optionally according to any of claims 1 to 6, the apparatus configured for use in forming a restriction in an organ of the gastrointestinal tract, optionally a stomach, the apparatus configured to be insertable into the interior of the organ, the apparatus comprising at least two, optionally at least three, optionally all of:
- an engagement device for engaging and manipulating organ wall tissue, to displace the tissue, optionally by pulling and/or folding and/or rolling the tissue;
- a scarring device for scarring tissue, optionally displaced tissue;
- a device for delivering a spacer and/or separator to tissue of the organ wall, optionally to displaced and/or engaged tissue, and/or tissue within operative range of the scarring device;
- a fixing device for inserting one or more tissue fixings optionally at a scarred zone of tissue.

8. A method of, or for use in, forming a restriction in an organ of the gastrointestinal tract, optionally a stomach, the method optionally using apparatus according to any of claims 1 to 7, the method comprising at least two, optionally at least three, optionally all of the following steps in any order:
- operating an engagement device to engaging and manipulate organ wall tissue, to displace the tissue, optionally by pulling and/or folding and/or rolling the tissue;
- operating a scarring device to scar tissue, optionally displaced tissue;
- operating a device to deliver a spacer and/or separator to tissue of the organ wall, optionally to displaced and/or engaged tissue, and/or tissue within operative range of the scarring device;
- operating a fixing device to insert one or more tissue fixings optionally at a scarred zone of tissue.
